# EUROPÄISCHE PATENTSCHRIFT

(11) **EP 0 853 628 B1**
(45) Veröffentlichungstag und Bekanntmachung des Hinweises auf die Patenterteilung: **02.10.2002**
(21) Anmeldenummer: 96927617.9
(22) Anmeldetag: 29.07.1996
(51) Int. Cl.: C07H 19/20

(54) **NEUE PHOTOLABILE 8-SUBSTITUIERTE CYCLISCHE NUCLEOTIDESTER, VERFAHREN ZU IHRER HERSTELLUNG UND IHRE VERWENDUNG**
PHOTOLABILE 8-SUBSTITUTED CYCLIC NUCLEOTIDE ESTERS, METHODS OF PREPARING THEM AND THEIR USE
NOUVEAUX ESTERS DE NUCLEOTIDES CYCLIQUES SUBSTITUES EN POSITION 8 ET PHOTOLABILES, PROCEDE DE PRODUCTION CORRESPONDANT ET UTILISATION DE CES COMPOSES

(30) Priorität: 28.07.1995 DE 19529025
(43) Veröffentlichungstag der Anmeldung: 22.07.1998
(73) Patentinhaber: Forschungsverbund Berlin e.V., 12489 Berlin (DE); Forschungszentrum Jülich GmbH, 52425 Jülich (DE)
(72) Erfinder: HAGEN, Volker, D-13051 Berlin (DE); KAUPP, Ulrich, Benjamin, D-52062 Aachen (DE)
(74) Vertreter: Gulde, Klaus W., Dipl.-Chem.
(86) Internationale Anmeldenummer: EP9603317
(87) Internationale Veröffentlichungsnummer: WO97005155

(56) Entgegenhaltungen:
- DE-A- 1 922 172
- US-A- 4 797 480
- NATURE, Bd. 310, 1984, Seiten 74-6, XP002019758 J.M. NERBONNE ET AL.: "New photoactivatable cyclic nucleotides produce intracellular jumps in cyclic AMP and cyclic GMP concentrations" in der Anmeldung erwähnt
- J. ORG. CHEM., Bd. 60, Nr. 13, 30.Juni 1995, Seiten 3953-6, XP002019759 M. IWAMURA ET AL.: "Photochemical Properties of New Photolabile cAMP Derivatives in a Physiological Saline Solution" in der Anmeldung erwähnt
- BIOCHEMISTRY, Bd. 35, Nr. 24, 18.Juni 1996, Seiten 7762-71, XP002019760 V. HAGEN ET. AL.: "Caged Compounds of Hydrolysis-Resistant Analogues of cAMP and cGMP"

## Beschreibung

Die Erfindung betrifft neue photolabile 8-substituierte cyclische Nucleotidester der Formel I 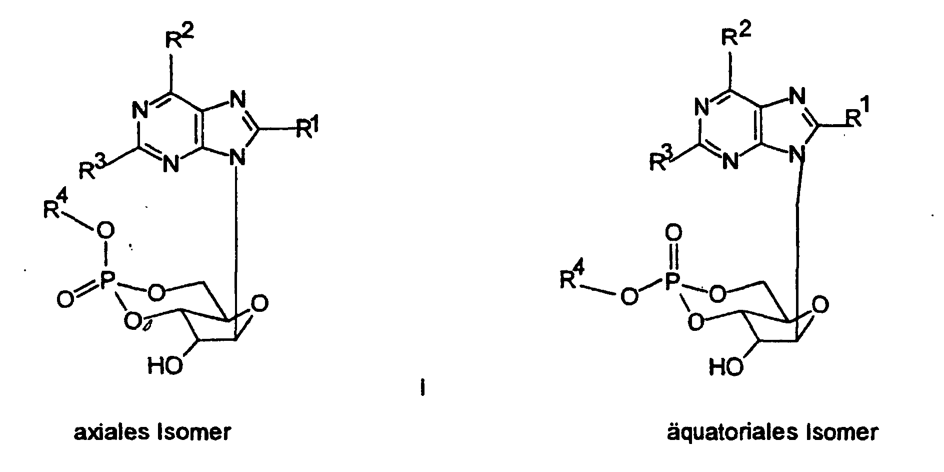 in der R¹ Brom, p-Chlor-phenylthio, (Fluorescein-5-ylcarbamoylmethylthio) oder (Fluorescein-6-ylcarbamoylmethylthio), R⁴ 4,5-Dialkoxy-2-nitro-benzyl, 1-(2-Nitro-phenyl)ethyl oder mono- oder dialkoxyliertes (Cumarin-4-yl)methyl und R² OH oder NH₂ und R³ NH₂ oder H bedeuten, wobei, wenn R² für OH steht, R³ NH₂ darstellen soll und, wenn R² für NH₂ steht, R³ H repräsentiert.

Die Verbindungen der Formel I, in denen R² OH bedeutet, liegen in der Dihydropyrimidonstruktur der Formel II vor. 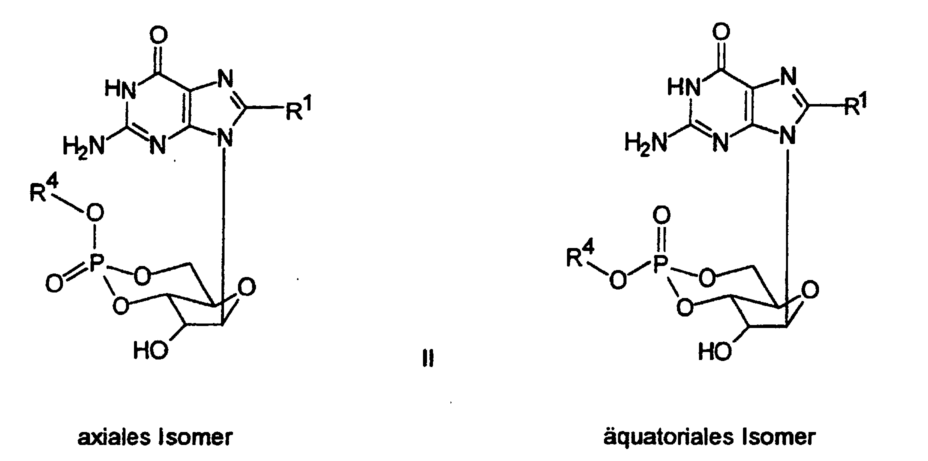 Ferner betrifft die Erfindung Verfahren zur Herstellung der Verbindungen der Formel I sowie deren Verwendung.

In der biologischen Forschung ist es bekannt, biologisch inaktive, photolabile Ester von natürlich vorkommenden cyclischen Nucleotiden nach Einführung in biologische Systeme durch Bestrahlung mit Licht in die biologisch aktiven cyclischen Nucleotide zu überführen, um auf diese Weise cyclisch nucleotid-abhängige Prozesse beeinflussen sowie zeitlich und mechanistisch untersuchen zu können [s. z. B. J. Nargeot, J. M. Nerbonne, J. Engels, H. A. Lester, Proc. Natl. Acad. Sci. USA 80, 2395 (1983); A. M. Gurney, H. A. Lester, Physiol. Rev. 67, 583 (1987); J. W. Karpen, A. L. Zimmermann, L. Stryer, D. A. Baylor, Proc. Natl. Acad. Sci. USA 85, 1287 (1988); J. A. McCray, D. R. Trentham, Annu. Rev. Biophys. Chem. 18, 239 (1989); J. E. T. Corrie; D. R. Trentham in Bioorganic Photochemistry Vol. 2: Biological Applications of Photochemical Switches (H. Morrison Ed.), 243, John Wiley & Sons (1993)].

Bisher wurden biologisch inaktive, photolabile Ester der cyclischen Nucleotide cyclisches Guanosin-3',5'-monophosphat (cGMP) und cyclisches Adenosin-3',5'-monophosphat (CAMP) für diese Zwecke eingesetzt. So wurden von J.M. Nerbonne et al. in Nature 310, 74 (1984) 4,5-Dimethoxy-2-nitrobenzylester sowohl von cAMP als auch von cGMP synthetisiert und beschrieben, die vor der Belichtung biologisch inaktiv sind und nach der Belichtung die biologisch aktiven cyclischen Nucleotide freigeben.

In der EP 0 233 403 ist ein 1-(2-Nitrophenyl)ethylester von cGMP und cAMP beschrieben.

T. Furuta et al. beschreiben in J. Org. Chemistry 60, 3953 (1995) einen (7-Methoxycumarin-4-yl)-methylester von cAMP, der nach Bestrahlung mit Licht von einer biologisch inaktiven Verbindung in eine biologisch aktive Verbindung überführt werden kann.

Beschrieben, aber bisher offenbar nicht in biologischen Experimenten eingesetzt, wurde auch der Desylester von cAMP [R. S. Givens, P. S. Athey, L. W. Kueper III, B. Matuszewski, J. Y. Xue, J. Am. Chem. Soc. 114, 8708 (1992); R. S. Givens, P. S. Athey, B. Matuszewski, L. W. Kueper III, J. Y. Xue, T. Fister, J. Am. Chem. Soc. 115, 6001 (1993)] sowie der (Anthrachinon-2-yl)methylund der (Naphth-2-yl)methylester von cAMP [T. Furuta, H. Torigai, M. Sugimoto, M. Iwamura, J. Org. Chem. 60, 3953 (1995)].
Bekannt sind entsprechende Synthesewege für diese Substanzen, die in biologischen Experimenten stets als Gemische der axialen und der äquatorialen Isomeren eingesetzt werden.

Am Purinrest chemisch modifizierte photolabile cGMPund cAMP-ester und ihre Verwendung zur Untersuchung cyclisch-nucleotid-abhängiger Prozesse sind bisher nicht beschrieben worden.
In der EP 0 044 527 wurden allerdings verschiedene in 8-Stellung des Purinrestes substituierte cAMP-Ester genannt. So. z.B. der 8-Brom- und der 8-(p-Chlorphenythio)-adenosin-3',5'-cyclophosphatbenzylester und weitere Nitro- und Methoxy-benzylester. Hinweise für deren Verwendung als photoaktivierbare Verbindungen werden jedoch nicht gegeben.

Weiterhin beschrieben worden sind die am Purinrest in 8-Stellung substituierten cyclische Nucleotide 8-Brom-cGMP und 8-Brom-cAMP (DE 19 22 172), 8-(p-Chlorphenylthio)-cGMP [J. P. Miller et al., Biochemistry 12, 5310 (1973)], 8-(p-Chlor-phenylthio)-cAMP, 8-(Fluorescein-5-yl-carbamoylmethylthio)- und 8-(Fluorescein-6-yl-carbamoylmethylthio)-cGMP und -cAMP und auch der p-Methyl-benzylester von 8-(Fluorescein-5-yl-carbamoylmethylthio)-cGMP [US-Pat. 4 797 480; J. C. Tanaka et al., Biochemistry 28, 2776 (1989)]. Alle zeigen keine photolabilen Eigenschaften.

Unbekannt sind bisher photolabile Ester von nichtnatürlichen, biologisch aktiven, cyclischen Nucleotiden mit Fluoreszenzeigenschaften.

Nachteile der bisher genutzten photolabilen cyclischen Nucleotidester sind, daß die daraus durch Photolyse freigesetzten natürlichen cyclischen Nucleotide cGMP und cAMP im allgemeinen erst in relativ hohen Konzentrationen ihre biologischen Effekte entfalten, wodurch der Einsatz relativ hoher Konzentrationen der photolabilen cyclischen Nucleotidester erforderlich ist, was aber teilweise durch deren unzureichende Löslichkeit in wäßrigen Lösungen begrenzt ist, und daß cGMP und cAMP schnell durch Phosphodiesterasen biologisch abgebaut werden.
Nachteilig für bestimmte Fragestellungen ist ferner teilweise die Solvolyseempfindlichkeit in wäßrigen Pufferlösungen und das Fehlen von Fluoreszenzeigenschaften der bekannten photolabilen Ester.

Der Erfindung liegt deshalb die Aufgabe zugrunde neue photolabile cyclische Nucleotidderivate für die Untersuchung cyclisch nucleotid-abhängiger Prozesse zu entwickeln, die bei der Photolyse nicht die natürlichen cyclischen Nucleotide cGMP und AMP, sondern biologisch hochaktive, phosphodiesteraseresistente cGMP- bzw. cAMP-Derivate freisetzen und die gegebenenfalls Fluoreszenzeigenschaften aufweisen sollen.

Die erfindungsgemäße Lösung der Aufgabe erfolgt mit neuen Verbindungen der allgemeinen Formel I, 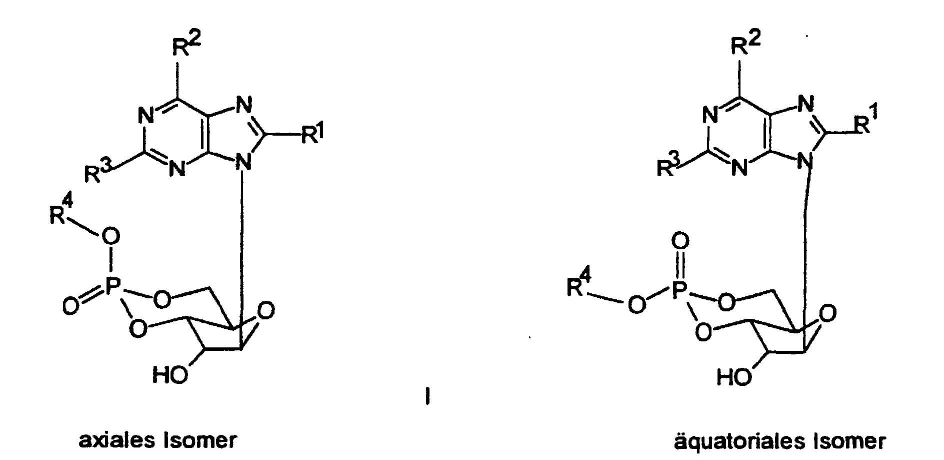 in der R¹ Brom, p-Chlor-phenylthio, (Fluorescein-5-ylcarbamoylmethylthio) oder (Fluorescein-6-ylcarbamoylmethylthio), R⁴ 4,5-Dialkoxy-2-nitro-benzyl, 1-(2-Nitro-phenyl)ethyl oder mono- oder dialkoxyliertes (Cumarin-4-yl)methyl und R² OH oder NH₂ und R³ NH₂ oder H bedeuten, wobei, wenn R² für OH steht, R³ NH₂ darstellen soll und, wenn R² für NH₂ steht, R³ H repräsentiert.

Die Verbindungen der Formel I, in denen R² OH und R³ NH₂ bedeuten, liegen in der Dihydropyrimidonstruktur der Formel II vor.

Die Herstellung der Verbindungen der Formel I erfolgt erfindungsgemäß zum einen durch Umsetzen von 8-Bromund 8-(p-Chlor-phenylthio)-cGMP bzw. 8-Brom- und 8-(p-Chlor-phenylthio)-cAMP mit Alkylierungsmitteln, z.B. entsprechend substituierten Phenyl- oder Cumarindiazoalkanen oder Phenylalkylhalogeniden bzw. niedrigalkoxylierten 4-Brommethyl-cumarinen und zum anderen durch Umsetzen der entsprechenden 8-Brom-cGMPund 8-Brom-cAMP-ester mit Thioharnstoff zu den entsprechenden Isothiuroniumbromiden und deren anschließende Reaktion mit Natriummethylat und 5- bzw. 6-Iod-acetamidofluorescein zu den mono- oder dialkoxylierten 2-Nitrobenzyl-, den mono- oder dialkoxylierten (Cumarin-4-yl)methyl- und den 1-(2-Nitrophenyl)ethylestern von 8-(Fluorescein-5-yl-carbamoylmethylthio)- und 8-(Fluorescein-6-yl-carbamoylmethylthio)-cGMP und -cAMP.

Im Ergebnis der Reaktion liegen die Verbindungen der Formel I als Isomerengemisch aus axialer und äquatorialer Form vor. Die Aufreinigung der Rohprodukte und die Trennung des Isomerengemisches wird chromatografisch durchgeführt. Hierbei hat sich als besonders vorteilhaft der Einsatz von flash-Chromatografie und/oder HPLC erwiesen.

Die gegenüber cGMP- und cAMP-Bindungsstellen weitgehend inaktiven Verbindungen der Formel I sind photolabil und lassen sich in wäßrigen Pufferlösungen oder nach Einführung in ein biologisches System durch Bestrahlung mit Licht in die freien biologisch wirksamen 8-substituierten cGMP-Derivate und die freien biologisch wirksamen 8-substituierten cAMP-Abkömmlinge überführen. Infolge der höheren biologischen Aktivität der dargestellten 8-substituierten cGMP und 8-substituierten cAMP-Derivate gegenüber cGMP und cAMP und einer weitgehenden Beständigkeit gegenüber Phosphodiesterasen, sind die neu hergestellten Verbindungen für die Untersuchung vieler cyclisch nucleotid-gesteuerter Vorgänge den bekannten photolabilen cGMP- bzw. cAMP-Derivaten weit überlegen. Insbesondere brauchen von den erfindungsgemäß hergestellten Verbindungen der Formel I gegenüber den bekannten photolabilen cyclischen Nucleotidestern zur Auslösung der gleichen Effekte wesentlich niedrigere Konzentrationen eingesetzt zu werden, und es entfallen alle mit der Phosphodiesteraseempfindlichkeit zusammenhängenden Nachteile.
Die neuen photolabilen cyclische Nucleotide ermöglichen erstmals die Untersuchung cyclisch nucleotid-gesteuerter Vorgänge, die hohe cGMP- bzw. cAMP-Konzentrationen erfordern, die infolge von Löslichkeitsproblemen bei den feststehenden Quantenausbeuten und Extinktionen der bekannten photolabilen cyclischen Nucleotide nicht erreichbar sind, und die Photolyse kann, infolge der höheren Aktivität der gebildeten freien cyclische Nucleotide, für gleiche Aktivierungen unter dem Einsatz von signifikant verringerter Lichtenergie erfolgen.
Der Einsatz niedrigerer Konzentrationen in biologischen Experimenten vermeidet oder mildert zusätzlich Komplikationen z. B. durch Diffusionsprobleme und toxische Wirkungen der eingesetzten Substanzen. Ein weitere Vorteil der 8-substituierten cGMP bzw. cAMP-Derivate ist ihre höhere Membrangängigkeit.

Die Phosphodiesteraseresistenz ermöglicht neue Zugänge zu mechanistischen Untersuchungen unter Ausschaltung der Phosphodiesterasen.

Infolge der Phosphodiesteraseresistenz der 8-substituierten cGMP- und cAMP-ester und der hohen Aktivität der freien cGMP- und cAMP-Derivate sind für die praktische Anwendung eine besonders hohe Reinheit und eine ausreichende Stabilität der photolabilen Ester gegenüber hydrolytischem Abbau in wäßrigen Puffersystemen erforderlich.
Erfindungsgemäß wird dies dadurch erreicht, daß vorzugsweise, insbesondere im Falle der relativ hydrolyseempfindlichen 4,5-Dimethoxy-2-nitrobenzylester, die reinen axialen Isomeren der entsprechenden cGMP- und cAMP-ester benutzt werden, die sich in wäßrigen Pufferlösungen, in denen sie in biologischen Experimenten üblicherweise eingesetzt werden, in allen Fällen als wesentlich hydrolysebeständiger als die äquatorialen Isomeren erwiesen haben. So liegt beispielsweise die Halbwertszeit der Hydrolyse in Acetonitril/ wäßrigem HEPES-Puffer für die axiale Form des 4,5-Dimethoxy-2-nitrobenzylesters von 8-Brom-cGMP 5 bis 10 mal höher als für die entsprechende äquatoriale Form.
Demgegenüber wurden für die äquatorialen Formen der 4,5-Dimethoxy-2-nitro-benzylester von cGMP und cAMP in wäßrigen Lösungen höhere Halbwertzeiten gefunden als für die entsprechenden axialen Formen [J. M. Nerbonne, S. Richard, J. Nargeot, H. A. Lester, Nature, 310, 74 (1984)].
Überraschenderweise erwiesen sich die axialen Formen der 4,5-Dimethoxy-2-nitrobenzylester von 8-Brom-cGMP und von 8-Brom-cAMP zusätzlich als wesentlich besser in wäßrigen Pufferlösungen löslich, wodurch sich ein weiterer Vorteil für den Einsatz dieser Verbindungen ergibt.
Die vorteilhafte Verwendung der reinen axialen Formen der cyclischen Nucleotide bei Untersuchungen in biologischen Systemen ist neu.

Die neuen photolabilen 8-substituierten cGMP- und cAMP-ester können für die Untersuchung cyclisch-nucleotidgesteuerter Vorgänge erfolgreich eingesetzt werden. Beispielsweise bewirken gegenüber dem 1-(2-Nitrophenyl)ethylester von cGMP wesentlich geringere Konzentrationen der axialen Form der im Beispiel 1 hergestellten Verbindung nach Photolyse eine vergleichbare Aktivierung des olfaktorischen cGMP-abhängigen Kationenkanals, wobei die Aktivierung infolge der Phosphodiesteraseresistenz der Verbindung nur sehr langsam abnimmt.

Darüberhinaus können die Verbindungen der Formel I als Ausgangsprodukte für die Darstellung weiterer in 8-Position strukturvariierter photoaktiver cGMP- und cAMP-Derivate genutzt werden. Beispielsweise lassen sich ausgehend von den 1-(2-Nitrophenyl)-ethyl-, den entsprechend niedrigalkoxylierten 2-Nitrobenzyl- und niedrigalkoxylierten (Cumarin-4-yl)methylestern von 8-Brom-cGMP und 8-Brom-cAMP über die Isothiuroniumsalze und nachfolgende Umsetzungen mit z. B. Iodacetamidofluoresceinen photoaktivierbare 1-(2-Nitrophenyl)ethyl- bzw. 4,5-Dimethoxy-2-nitrobenzylester und mono- oder dialkoxylierte (Cumarin-4-yl)methylester von 8-(Fluorescein-5-yl-carbamoylmethylthio)- und 8-(Fluorescein-6-yl-carbamoylmethylthio)-cGMP und -cAMP herstellen. Dieses Verfahren ist besonders für solche Fälle interessant, in denen der Substituent in 8-Position reaktionsfähige Gruppen aufweist, die mit Diazoalkanen bzw. Bromalkanen reagieren können.

Bei Einsatz radioaktiv markierter Ausgangsverbindungen lassen sich radioaktiv markierte photoaktive Verbindungen der Formel I darstellen.
Die hochwirksamen und fluorescenzaktiven Verbindungen der Formel I sind vorteilhafterweise leicht quantitativ und qualitativ erfaßbar.

### Ausführungsbeispiele

### Beispiel 1

### 8-Brom-cGMP-4, 5-dimethoxy-2-nitrobenzylester

Eine Mischung aus 0,4 mmol 8-Brom-cGMP und etwa 3 mmol frisch hergestelltes 4,5-Dimethoxy-2-nitro-phenyldiazomethan [J. F. Wooton, D. R. Trentham in Photochemical Probes in Biochemistry, NATO ASI Ser. C, Vol. 272, 277 (1989)] in 15 ml DMSO wird unter Lichtausschluß 40 Stunden bei Raumtemperatur gerührt. DMSO und einige Nebenprodukte werden durch mehrmaliges Ausschütteln mit Ether entfernt und der Rückstand mittels flash Chromatografie (Elution mit Chloroform/Methanol) oder präparative HPLC (PLRP-S, 10 µm, 250 x 25 mm i.d., Flußgeschwindigkeit 10 ml/Min., linearer Gradient 20%-60% B in 70 Min.,Eluent A: Wasser, Eluent B: Acetonitril) gereinigt und gegebenenfalls gleichzeitig in das axiale und das äquatoriale Isomer aufgetrennt.
Ausbeute: 45 % d. Theorie.
C₁₉H₂₀Br₁N₆O₁₁P₁ x 2H₂O (655,32) aus Acetonitril/Wasser nach Lyophilisierung.
**axiales Isomer:** ³¹P-NMR (DMSO-d₆): δ -5,17; DC (Chloroform/Methanol 5:1): R_{f}-Wert: 0,60.
**äquatoriales Isomer:** ³¹P-NMR (DMSO-d₆): δ -4,27; DC (Chloroform/Methanol 5:1): R_{f}-Wert: 0,54.

### Beispiel 2

### 8-Brom-cGMP-1- (2-nitro-phenyl)ethylester

Zu 0,4 mmol 8-Brom-cGMP in 15 ml DMSO werden unter Rühren ca. 3mmol frisch in Chloroform hergestelltes 1-(2-Nitro-phenyl)diazoethan [J. W. Walker, G. P. Reid, D. R. Trentham, Methods in Enzymology (S. Fleisher und B. Fleisher Eds.) Vol 172, 288 (1989)] gegeben. Chloroform wird bei Raumtemperatur abrotiert und anschließend wird 30 Stunden bei Raumtemperatur unter Lichtausschluß gerührt. DMSO und einige Nebenprodukte werden durch wiederholtes Ausschütteln mit Ether entfernt und der Rückstand mittels flash Chromatografie oder HPLC gereinigt und gegebenenfalls gleichzeitig in das axiale und das äquatoriale Isomer aufgetrennt.
Ausbeute: 51 % d. Theorie.
C₁₈H₁₈Br₁N₆O₉P₁ x H₂O (591,28) aus Acetonitril/Wasser nach Lyophilisierung.
**Diastereoisomerenpaar der axialen Form:** ³¹P-NMR (DMSOd₆): δ -5,91 und -6,02; DC (Chloroform/Methanol 5:1): R_{f}-Wert: 0,64 bzw. 0,68.
**Diastereoisomerenpaar der äquatorialen Form:** ³¹P-NMR (DMSO-d₆): δ -4,59 und -4,76; DC (Chloroform/Methanol 5:1): R_{f}-Wert: 0,70.

### Beispiel 3

### 8-Brom-cAMP-(7-methoxy-cumarin-4-yl)methylester

Eine Mischung aus 0,25 mmol 8-Br-cAMP, 0,75 mmol 4-Brommethyl-7-methoxy-cumarin und 0,5 mmol Silber(I)-oxid wird in Anlehnung an ein von T. Furuta et al. (J. Org. Chemistry 60, 3953 (1995) beschriebenes Verfahren in 15 ml Acetonitril und 2,5 ml DMSO 50 Stunden bei 60°C gerührt. Die gebildete Suspension wird anschließend heiß filtriert, der Rückstand mit Chloroform ausgewaschen und die vereinigten Filtrate am Rotationsverdampfer zur Trockne eingeengt. Der Rückstand wird mehrfach mit Ether ausgezogen und mittels flash Chromatografie oder präparative HPLC gereinigt und gegebenenfalls gleichzeitig in das axiale und das äquatoriale Isomer aufgetrennt.
Ausbeute: 34% d. Theorie.
C₂₁H₁₉N₅O₉P₁Br₁·H₂O (614,3) aus Acetonitril/Wasser nach Lyophilisierung
**axiales Isomer:** ³¹P-NMR (DMSO-d₆): δ -5,10; DC (Chloroform/Methanol 5:1): R_{f}-Wert:0,66
**äquatoriales Isomer:** ³¹P-NMR (DMSO-d₆): δ -3,43; DC (Chloroform/Methanol 5:1): R_{f}-Wert:0,63

### Beispiel 4

### 8-Brom-cAMP-4,5-dimethoxy-2-nitrobenzylester

Analog Beispiel 1 aus 0.4 mmol 8-Br-cAMP und 3 mmol 4,5-Dimethoxy-2-nitrophenyldiazomethan.
Ausbeute: 51% d. Theorie.
C₁₉H₂₀Br₁N₆O₁₀P₁ x H₂O (621,3) aus Acetonitril/Wasser nach Lyophilisierung.
**axiales Isomer:** ³¹P-NMR (DMSO-d₆): δ -5,19; DC (Chloroform/Methanol 5:1): R_{f}-Wert: 0,71.
**äquatoriales Isomer:** ³¹P-NMR (DMSO-d₆): δ -3,37; DC (Chloroform/Methanol 5:1): R_{f}-Wert: 0,61.

### Beispiel 5

### 8-Brom-cAMP-1-(2-nitro-phenyl)ethylester

Analog Beispiel 2 aus 0,4 mmol 8-Brom-cAMP und 3 mmol 1-(2-Nitro-phenyl)diazoethan und 40 Stunden Rühren bei Raumtemperatur.
Ausbeute: 57 % d. Theorie.
C₁₈H₁₈Br₁N₆O₈P₁ x H₂O (575,28) aus Acetonitril/Wasser nach Lyophilisierung
**Diastereoisomerenpaar der axialen Form:** ³¹P-NMR (DMSOd₆) : δ -5,98 und -6,07; DC (Chloroform/Methanol 5:1): R_{f}-Wert: 0,68.
**Diastereoisomerenpaar der äquatorialen Form:** ³¹P-NMR (DMSO-d₆): δ -3,65 und -3,89 ; DC (Chloroform/Methanol 5:1): R_{f}-Wert: 0,58.

### Beispiel 6

### 8-Brom-cGMP-(7-methoxy-cumarin-4-yl)methylester

Analog Beispiel 3 aus 0,4 mmol 8-Brom-cAMP und 1,2 mmol 4-Brommethyl-7-methoxy-cumarin und 0,5 mmol Silber(I)-oxid in 15 ml Acetonitril und 5 ml DMSO und 60 Stunden Erwärmen.

### Beispiel 7

### 8-(4-Chlorphenylthio)-cGMP-4,5-dimethoxy-2-nitrobenzylester

Analog Beispiel 1 aus 0.4 mmol 8-(4-Chlorphenylthio)-cGMP und 3 mmol 4,5-Dimethoxy-2-nitrophenyldiazomethan.
Ausbeute: 68% d. Theorie.
C₂₅H₂₄Cl₁N₆O₁₁P₁S₁ · H₂O (701,0) aus Acetonitril/Wasser nach Lyophilisierung.
**axiales Isomer:** ³¹P-NMR (DMSO-d₆): δ -5,12. DC (Chloroform/Methanol 5:1): R_{f}-Wert: 0,69.
**äquatoriales Isomer:** ³¹P-NMR (DMSO-d₆): δ -4,31. DC (Chloroform/Methanol 5:1): R_{f}-Wert: 0,62.

### Beispiel 8

**8-(4-Chlorphenylthio)-cGMP-1-(2-nitro-phenyl)ethylester** Analog Beispiel 2 aus 0,4 mmol 8-(4-Chlorphenylthio)-cGMP und 3 mmol 1-(2-Nitro-phenyl)ethyldiazoethan und 40 Stunden Rühren bei Raumtemperatur.

### Beispiel 9

### 8- (4-Chlorphenylthio)-cGMP-(7-methoxy-cumarin-4-yl)methylester

Analog Beispiel 3 aus 0,4 mmol 8-(4-Chlorphenylthio)-cGMP und 1,2 mmol 4-Brommethyl-7-methoxy-cumarin und 0,8 mmol Silber(I)-oxid in 24 ml Acetonitril und 8 ml DMSO.
Ausbeute: 15% d. Theorie.
C₂₇H₂₃Cl₁N₅O₁₀P₁S₁ (675,99) Acetonitril/Wasser nach Lyophilisierung
axiales Isomer: DC (Chloroform/Methanol 5:1): R_{f}-Wert:0,59
äquatoriales Isomer: DC (Chloroform/Methanol 5:1): R_{f}-Wert:0,57

### Beispiel 10

### 8-(4-Chlorphenylthio)-cAMP-4,5-dimethoxy-2-nitrobenzylester

Analog Beispiel 1 aus 0.4 mmol 8-(4-Chlorphenylthio)-cAMP und 3 mmol 4,5-Dimethoxy-2-nitrophenyldiazomethan.

### Beispiel 11

### 8-(4-Chlorphenylthio)-cAMP-1-(2-nitro-phenyl)ethylester

Analog Beispiel 2 aus 0.4 mmol 8-(4-Chlorphenylthio)-cAMP und 3 mmol 1-(2-Nitrophenyl)ethyldiazoethan und 40 Stunden Rühren bei Raumtemperatur.
Ausbeute: 38% d. Theorie.
C₂₄H₂₂Cl₁N₆O₈P₁ S₁ (620,96) aus Acetonitril/Wasser nach Lyophilisierung.
**Diastereoisomerenpaar der axialen Form:** ³¹P-NMR (DMSOd₆): δ - 5,94 und -6,08; DC (Chloroform/Methanol 5:1): R_{f}-Wert: 0,82.
**Diastereoisomerenpaar der äquatorialen Form:** ³¹P-NMR (DMSO-d₆): δ -3,63 und -3,85; DC (Chloroform/Methanol 5:1): Rf-Wert: 0,71.

### Beispiel 12

### 8-(4-Chlorphenylthio)-cAMP-(7-methoxy-cumarin-4-yl)methylester

Analog Beispiel 3 aus o,4 mmol 8-(4-Chlorphenylthio)-cAMP, 1,2 mmol 4-Brommethyl-7-methoxy-cumarin und 0,8 mmol Silber(I)oxid.

### Beispiel 13

### 8-(Fluorescein-5-yl-carbamoylmethylthio)-cGMP-1-(2-nitrophenyl)ethylester

59,1 mg (0,1 mmol) des nach Beispiel 2 hergestellten 1-(2-Nitro-phenyl)ethylesters von 8-Brom-cGMP (Gemisch der axialen und der äquatorialen Form) werden mit 30,45 mg (0,4mmol) Thioharnstoff in 4 ml Methanol 1 Stunde am Rückfluß gekocht. Es wird abgekühlt, das gebildete Isothiuroniumbromid mit Ether gefällt, abgesaugt, in 15 ml Methanol gelöst, mit 30 mg Natriummethylat versetzt, mit 40 mg 5-Iodacetamidofluorescein 1 Stunde bei Raumtemperatur gerührt, am Rotationsverdampfer einrotiert und anschließend mittels präparativer HPLC gereinigt bzw gegebenenfalls in das axiale und äquatoriale Isomere getrennt.
Ausbeute: 25% d. Theorie.
**Diastereoisomerenpaar der axialen Form:** DC (Butanol-/Aceton/Wasser/Essigsäure/konz. NH₃ = 35:25:25:14,2: 0,8): R_{f}-Wert: 0,82. **Diastereoisomerenpaar der äquatorialen Form:** DC (Butanol/Aceton/Wasser/Essigsäure/konz. NH₃ = 5:25:25:14,2:0.8): R_{f}-Wert: 0,77.

### Beispiel 14

### 8-(Fluorescein-5-yl-carbamoylmethylthio)-cGMP-1-(2-nitrophenyl)ethylester, axiales Isomer

Analog Beispiel 13, aber ausgehend von der axialen Form des 1-(2-Nitrophenyl)ethylesters von 8-Brom-cGMP.

### Beispiel 15

### 8-(Fluorescein-5-yl-carbamoylmethylthio)-cGMP-4,5-dimethoxy-2-nitrobenzylester

Analog Beispiel 13 ausgehend von dem in Beispiel 1 hergestellten 4,5-Dimethoxy-2-nitrobenzylester von 8-Brom-cGMP.

### Beispiel 16

### 8-(Fluorescein-5-yl-carbamoylmethylthio)-cGMP-(7-methoxy-cumarin-4-yl)methylester

Analog Beispiel 13 ausgehend von dem in Beispiel 6 hergestellten (7-Methoxy-cumarin-4-yl)methylester von 8-Brom-cGMP

### Beispiel 17

### 8-(Fluorescein-5-yl-carbamoylmethylthio)-cAMP-1-(2-nitrophenyl)ethylester

Analog Beispiel 13 ausgehend von dem in Beispiel 5 hergestellten 1-(2-Nitrophenyl)ethylester von 8-Brom-cAMP.

### Beispiel 18

### 8-(Fluorescein-5-yl-carbamoylmethylthio)-cAMP-4,5-dimethoxy-2-nitrobenzylester

Analog Beispiel 13 ausgehend von dem in Beispiel 4 hergestellten 4,5-Dimethoxy-2-nitrobenzylester von 8-Brom-cAMP.

### Beispiel 19

### 8-(Fluorescein-5-yl-carbamoylmethylthio)-cAMP-4,5-dimethoxy-2-nitrobenzylester, axiales Isomer

Analog Beispiel 13 ausgehend von der axialen Form des 4,5-Dimethoxy-2-nitrobenzylesters von 8-Brom-cAMP.

### Beispiel 20

### 8-(Fluorescein-5-yl-carbamoylmethylthio)-cAMP-(7-methoxy-cumarin-4-yl)-methylester

Analog Beispiel 13 ausgehend von dem in Beispiel 3 hergestellten (7-Methoxy-cumarin-4-yl)methylester von 8-Brom-cAMP.

### Beispiel 21

### Aktivierung des olfaktorischen CNG-Kanals durch photolytische Freisetzung von 8-Brom-cGMP bzw. von cGMP

In Whole-Cell-Messungen an HEK293-Zellen wurde elektrophysiologisch die Aktivierung der heterolog exprimierten α-Untereinheit des olfaktorischen CNG-Kanals durch photolytische Freisetzung von cGMP aus dem 1-(2-Nitrophenyl)ethylester von cGMP (Calbiochem) und dem 8-Brom-cGMP aus der axialen Form des 4,5-Dimethoxy-2-nitrophenylesters von 8-Brom-cGMP vergleichend untersucht. Hierbei ergaben sich bei Einsatz einer intrazellulären Lösung von 75 µM 1-(2-Nitro-phenyl)ethyl-cGMP und einer intrazellulären Lösung von 6,25 µM (4,5-Dimethoxy-2-nitro-benzyl)-8-brom-cGMP und gleichartiger Belichtung mit UV-Licht (Lichtblitze 10 bis 500 ms, 100 W Hg-Kurzbogenlampe mit 335 nm cutoff-Filter) vergleichbare Einwärtsionenströme, d.h. analoge Aktivierungen des Kanals. Die Verfolgung des zeitlichen Stromverlaufs zeigt nur bei der photolytischen Freisetzung von cGMP eine schnelle Abnahme, die durch den Abbau durch Phosphodiesterasen bedingt ist.

## Patentansprüche

1. Photolabile 8-substituierte cyclische Nucleotidester der Formel I 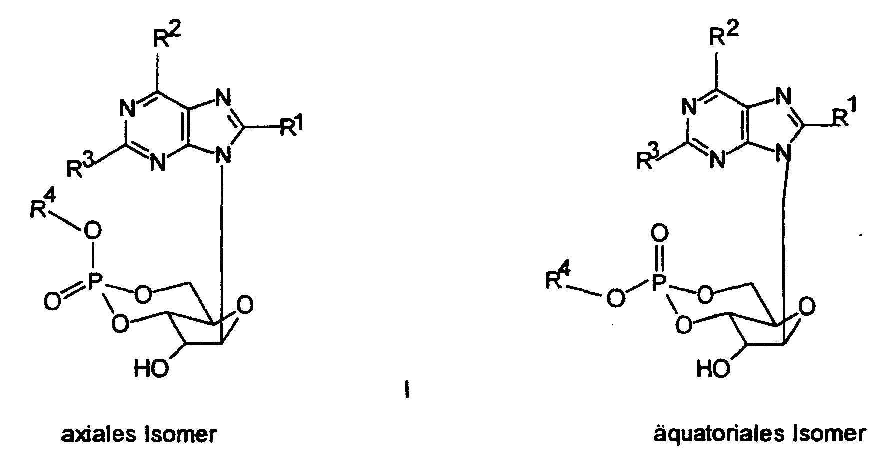 in der R¹ Brom, p-Chlor-phenylthio, (Fluorescein-5-yl-carbamoylmethylthio) oder (Fluorescein-6-ylcarbamoylmethylthio), R⁴ 4,5-Dialkoxy-2-nitro-benzyl, 1-(2-Nitro-phenyl)ethyl oder mono- oder dialkoxyliertes (Cumarin-4-yl)methyl und R² OH oder NH₂ und R³ NH₂ oder H bedeuten, wobei, wenn R² für OH steht, R³ NH₂ darstellen soll und, wenn R² für NH₂ steht, R³ H repräsentiert.

2. Verfahren zur Herstellung von Verbindungen gemäß Anspruch 1,
**gekennzeichnet dadurch,**
**daß** 8-Brom-cGMP, 8-Brom-cAMP, 8-(p-Chlorphenylthio)-cGMP oder 8-(p-Chlor-phenylthio)-cAMP mit entsprechend substituierten Diazoalkanen oder Bromalkanen umgesetzt werden.

3. Verfahren zur Herstellung von Verbindungen gemäß Anspruch 1,
**gekennzeichnet dadurch,**
**daß** niedrigalkoxylierte 2-Nitrobenzyl-, niedrigalkoxylierte (Cumarin-4-yl)methyl- und 1-(2-Nitro-phenyl)ethylester von 8-Brom-cGMP und 8-Brom-cAMP mit Thioharnstoff in die Isothiuroniumsalze überführt und anschließend mit Natriummethylat und 5- oder 6-Iod-acetamido-fluorescein zur Reaktion gebracht werden.

4. Verfahren nach Anspruch 2 oder 3,
**gekennzeichnet dadurch,**
**daß** eine Aufreinigung der Rohprodukte und eine Isomerentrennung chromatografisch wie durch flash-Chromatografie oder HPLC erfolgt.

5. Verwendung der Verbindungen gemäß Anspruch 1 zur photochemischen Freisetzung von biologisch aktivem 8-Brom-cGMP, 8-Brom-cAMP, 8-(p-Chlor-phenylthio)-cGMP, 8-(p-Chlor-phenylthio)-cAMP, 8-(Fluorescein-5-yl-carbamoyl-methylthio)-cGMP, 8-(Fluorescein-5-yl-carbamoyl-methylthio)-cAMP, 8-(Fluorescein-6-ylcarbamoyl-methylthio)-cGMP oder 8-(Fluorescein-6-yl-carbamoylmethylthio)-cAMP.

6. Verwendung der Verbindungen gemäß Anspruch 1 zur Beeinflussung und zur Untersuchung cyclisch-nucleotid-abhängiger Prozesse in biologischen Systemen.

7. Verwendung der reinen axialen Isomere gemäß Anspruch 1 zur Beeinflussung und zur Untersuchung cyclisch nucleotid-abhängiger Prozesse in biologischen Systemen.

8. Verwendung der fluoreszenzaktiven 8-(Fluorescein-5-yl-carbamoylmethylthio)- und 8-(Fluorescein-6-ylcarbamoylmethylthio)-cGMP- und -cAMP-ester sowie der (Cumarin-4-yl)methyl-cGMP- und -cAMP-ester nach Anspruch 1 zur Beeinflussung und zur Untersuchung cyclisch-nucleotid-abhängiger Prozesse in biologischen Systemen.

## Claims

1. Photolabile 8-substituted cyclic nucleotide esters of formula I 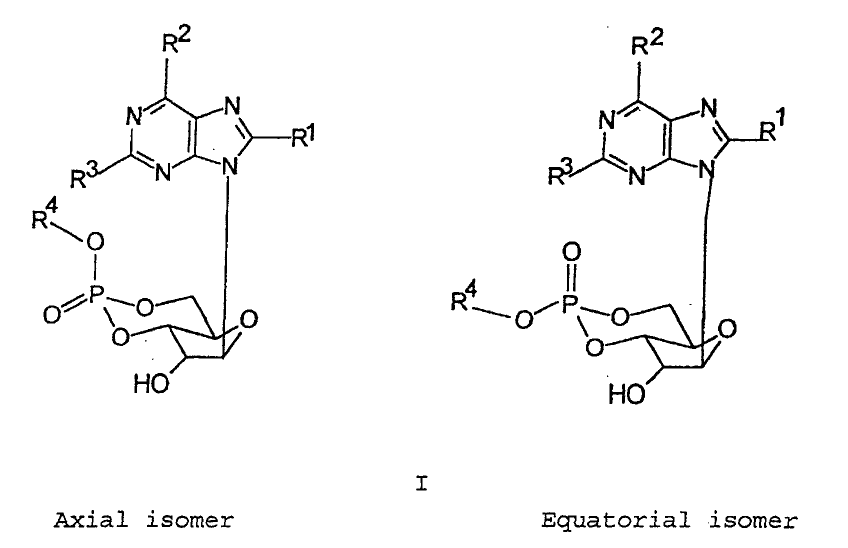 wherein R¹ represents bromine, p-chlorophenylthio, (fluorescein-5-ylcarbamylmethylthio) or (fluorescein-6-ylcarbamylmethylthio), R⁴ represents 4,5-dialkoxy-2-nitrobenzyl, 1-(2-nitrophenyl)ethyl or mono- or dialkoxylated (coumarin-4-yl)methyl, and R² represents OH or NH₂, and R³ represents NH₂ or H, and if R² represents OH, R³ is to be NH₂, and, if R² represents NH₂, R³ is H.

2. A method for preparing the compounds according to claim 1, **characterized by** reacting 8-bromo-cGMP, 8-bromo-cAMP, 8-(p-chlorophenylthio)-cGMP, or 8-(p-chlorophenylthio)-cAMP with appropriately substituted diazoalkanes or bromoalkanes.

3. A method for preparing the compounds according to claim 1, **characterized in that** lower-alkoxylated 2-nitrobenzyl, lower-alkoxylated (coumarin-4-yl)methyl and 1-(2-nitrophenyl)ethyl esters of 8-bromo-cGMP and 8-bromo-cAMP are converted to the isothiuronium salts using thiourea and subsequently reacted with sodium methylate and 5- or 6-iodoacetamidofluorescein.

4. The method according to claim 2 or 3, **characterized in that** purification of the crude products and separation of the isomers is effected using chromatography, such as flash chromatography or HPLC.

5. Use of the compounds according to claim 1 in the photochemical liberation of biologically active 8-bromo-cGMP, 8-bromo-cAMP, 8-(p-chlorophenylthio)-cGMP, 8-(p-chlorophenylthio)-cAMP, 8-(fluorescein-5-yl-carbamylmethylthio)-cGMP, 8-(fluorescein-5-ylcarbamylmethylthio)-cAMP, 8-(fluorescein-6-ylcarbamylmethylthio)-cGMP, or 8-(fluorescein-6-ylcarbamylmethylthio)-cAMP.

6. Use of the compounds according to claim 1 for controlling and investigating cyclic nucleotide-dependent processes in biological systems.

7. Use of the pure axial isomers according to claim 1 for controlling and investigating cyclic nucleotide-dependent processes in biological systems.

8. Use of the fluorescence-active 8-(fluorescein-5-ylcarbamylmethylthio)- and 8-(fluorescein-6-ylcarbamylmethylthio)-cGMP and cAMP esters and the (coumarin-4-yl)methyl-cGMP and cAMP esters according to claim 1 for controlling and investigating cyclic nucleotide-dependent processes in biological systems.

## Revendications

1. Ester de type nucléotide cyclique photolabile 8-substitué avec la formule suivante : 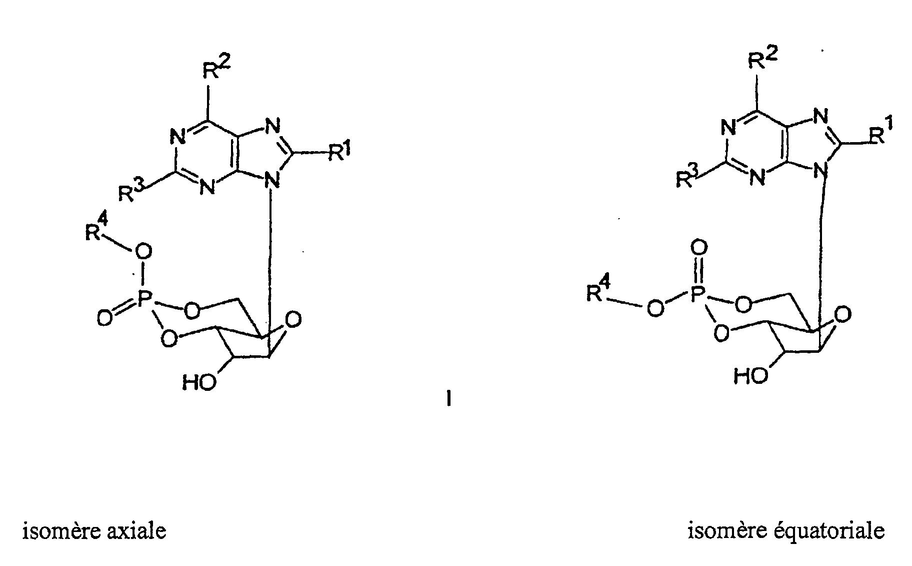 dans lequel signifie R¹ brome, p-chlore-phénylthio, (fluorescéine-5-yl-carbamoylmethylthio) ou (fluorescéine-6-yl-carbamoylmethylthio), R⁴ signifie 4,5 dialkoxy-2-nitro-benzyl, 1-(2-nitro-phenyl)éthyl ou (coumarine-4-yl)méthyl mono- ou di-alkoxylé et R² signifie OH ou NH₂ et R³ signifie NH₂ ou H, où , si R² signifie OH, R³ signifie NH₂ et si R² signifie NH₂, R³ signifie H.

2. Procédé pour la fabrication de liaisons selon la revendication 1,
**caractérisé en ce qu'**on fait réagir 8-brome-cGMP, 8-brome-cAMP, 8-(p-chlore-phénylthio)-cGMP où 8-(p-chlore-phénylthio)-cAMP avec des diazoalcanes ou des bromealcanes substitués de façon appropriée.

3. Procédé pour la fabrication de liaisons selon la revendication 1,
**caractérisé en ce que** des ester éthyliques de 2-nitrobenzyle faiblement alkoxylés , de (coumarine-4-yl) méthyle faiblement alkoxylés et de 1-(2-nitro-phényle) , de 8-brome-cGMP et de 8-brome-cAMP sont transformés avec de la thiourée en sels de isothiuronium et qu'on fait réagir ces sels avec du méthylate de sodium et 5- ou 6- iode-acétamido-fluorescéine.

4. Procédé selon la revendication 2 ou 3, **caractérisé en ce qu'**un nettoyage des produits brutes et une séparation des isomères est effectué de manière chromatographique comme par exemple la chromatographie flash ou HPLC.

5. Utilisation des liaisons selon la revendication 1, pour une libération photochimique de 8-brome-cGMP, 8-brome-cAMP, 8-(p-chlore-phénylethio)-cGMP, 8-(p-chlore-phénylethio)-cAMP, 8- (fluorescéine-5-yl-carbamoyl-methylthio)-cGMP, 8-(fluorescéine-5-yl-carbamoylmethylthio)-cAMP, 8- (fluorescéine-6-yl-carbamoylmethylthio)-cGMP ou 8-(fluorescéine-6-yl-carbamoyl-methylthio)-cAMP biologiquement actifs.

6. Utilisation des liaisons de la revendication 1 pour influencer et examiner dans des systèmes biologiques des processus pilotés par des nucléotides cycliques.

7. Utilisation des isomères axiales pures selon la revendication 1 pour influencer et examiner dans des systèmes biologiques des processus pilotés par des nucléotides cycliques.

8. Utilisation des esters de 8-(fluorescéine-5-yl-carbamoyl-methylthio)- et 8- (fluorescéine-6-yl-carbamoylmethylthio)-cGMP et -cAMP florescents ainsi que des esters de (coumarine-4y1)-méthyle-cGMP et -cAMP pour influencer et examiner dans des systèmes biologiques des processus pilotés par des nucléotides cycliques.
